# EUROPEAN PATENT APPLICATION

(11) **EP 4 792 799 A1**
(43) Date of publication of application: **19.08.2026**
(21) Application number: 25157750.8
(22) Date of filing: 13.02.2025
(51) Int. Cl.: A61J 7/00, G01N 21/95, G07F 11/42, B65B 9/08

(54) **ARRANGEMENT FOR HANDLING A STRIP OF MEDICATION SACHETS**

(71) Applicant: Evondos Oy, 24240 Salo (FI)
(72) Inventor: JOKINEN, Juuso, 03100 Nummela (FI); ENARVI, Timo, 00250 Helsinki (FI); VUORENMÄKI, Antero, 01800 Klaukkala (FI); JONES, Steven, SG8 5EU Hertfordshire (GB); NEWMAN, Matthew, CB24 3BB Cambridgeshire (GB); CRICHTON, Daniel, CB4 1HE Cambridge (GB); MAUND, Alexander, KT13 8LB Surrey (GB); MACLACHLAN, Alex, IP14 4JX Suffolk (GB); GRIMWADE, Steve, PE12 0TU Lincolnshire (GB)
(74) Representative: Berggren Oy

(57) **Abstract**

The present invention relates to an arrangement for handling a strip (100) of medication sachets (101). The arrangement comprises a conveying device (200, 600) for conveying the strip (100) of medication sachets (101), the conveying device (200, 600) comprising an inlet (201, 610) for receiving the medication sachets (101), an outlet (202, 614) for discharging the medication sachets (101) and means (203, 204, 205, 206, 601, 602, 603, 604, 605) for conveying the medication sachets (101) from the inlet (201, 610) to the outlet (202, 614), a first imaging device (300) arranged in connection with the inlet (201, 610) and configured to capture at least one image of the medication sachet (101) before it is conveyed to the inlet (201, 610), and a second imaging device (400) arranged in connection with the outlet (202, 614) and configured to capture at least one image of the medication sachet (101) after it has been conveyed out of the outlet (202, 614). The present invention also relates to a medication dispenser (500).

## Description

### TECHNICAL FIELD OF THE INVENTION

The present invention relates to an arrangement for handling a strip of medication sachets according to the preamble of the appended independent claim. The invention also relates to a medication dispenser.

### BACKGROUND OF THE INVENTION

Various medication dispensers are known for automated dispensing of medications to a patient. Many of these medication dispensers use strips of prepackaged medication sachets, where each medication sachet contains the medications that the patient should take at a prescribed date and time.

A conventional medication dispenser comprises a chamber for a strip of medication sachets to be dispensed, and one or more conveying devices for conveying the medication sachets from the chamber through an imaging device and a cutting device to an outlet from which a patient can take the medication sachets. The imaging device is used for imaging the medication sachets for the purpose of extracting information from the medication sachets, such as patient's name, and dispense date and time, and/or for detecting seam areas between medication sachets. The cutting device is used for separating the medication sachets from the strip of medication sachets by cutting the strip in a transverse direction along the detected seam areas.

A problem associated with the known medication dispensers relates to situations where medication sachets to be dispensed are running out, or where a wrong strip of medication sachets has been inserted into the medication dispenser. In the known medication dispensers, after either of these situations has been detected, there is only very little time to insert a new strip of medication sachets into the medication dispenser so that the dispensing of medications can be continued as intended.

### OBJECTIVES OF THE INVENTION

It is the main objective of the present invention to reduce or even eliminate the prior art problems presented above.

It is an objective of the present invention to provide an arrangement for handling a strip of medication sachets in a medication dispenser. In more detail, it is an objective of the invention to provide an arrangement that gives more time to insert a new strip of medication sachets into a medication dispenser in situations where it has been detected that medication sachets are running out, or a wrong strip of medication sachets has been inserted into the medication dispenser.

It is also an objective of the present invention to provide a medication dispenser for dispensing medication sachets to a patient.

In order to realise the above-mentioned objectives, the arrangement according to the invention is characterised by what is presented in the characterising portion of the appended independent claim. Advantageous embodiments of the invention are described in the dependent claims.

### DESCRIPTION OF THE INVENTION

An arrangement for handling a strip of medication sachets according to the invention comprises a conveying device for conveying the strip of medication sachets, the conveying device comprising an inlet for receiving the medication sachets, an outlet for discharging the medication sachets and means for conveying the medication sachets from the inlet to the outlet, a first imaging device arranged in connection with the inlet and configured to capture at least one image of the medication sachet before it is conveyed to the inlet, and a second imaging device arranged in connection with the outlet and configured to capture at least one image of the medication sachet after it has been conveyed out of the outlet.

The arrangement according to the invention can be used in a medication dispenser for handling a strip of medication sachets. The strip of medication sachets comprises medication sachets attached one to another, where each medication sachet contains the medications that a patient should take at a prescribed date and time.

The arrangement according to the invention comprises two imaging devices and the conveying device between them. The first imaging device images the medication sachets before they are conveyed with the conveying device to the second imaging device that images the conveyed medication sachets. The first imaging device is configured to capture at least one image of the medication sachet. The at least one image captured with the first imaging device can be processed to determine patient and medication related information of the medication sachet, such as a patient identifier (e.g. name and/or identification number), and dispense date and time. This information enables detecting situations where medication sachets are running out, or a wrong strip of medication sachets has been inserted into the medication dispenser. The at least one image captured with the first imaging device can alternatively be processed to detect an end of the strip of medication sachets, i.e. the last medication sachet. The second imaging device is configured to capture at least one image of the medication sachet. The at least one image captured with the second imaging device can be processed to detect a seam area between two medication sachets. The first and the second imaging device comprises a camera that is preferably a digital camera. The first and the second imaging device may also comprise other optical components.

The medication sachets are received one after another into the conveying device through the inlet. From the inlet the medication sachets are conveyed with the conveying means to the outlet through which the medication sachets are discharged out of the conveying device. The conveying means may comprise, for example, one or more pairs of foam rollers and/or opposed belt conveyors through which the strip of medication sachets is conveyed. The conveying means can convey a plurality of medication sachets simultaneously, which means that the conveying device can store medication sachets while conveying them. Preferably, the means for conveying the medication sachets from the inlet to the outlet can simultaneously convey at least three medication sachets.

An advantage of the arrangement according to the invention is that it gives more time to insert a new strip of medication sachets into a medication dispenser in situations where it has been detected that medication sachets are running out, or a wrong strip of medication sachets has been inserted into the medication dispenser.

According to an embodiment of the invention the arrangement comprises means for processing the at least one image captured with the first imaging device to determine patient and medication related information of the medication sachet. The patient and medication related information may contain a patient identifier (e.g. name and/or identification number), and dispense date and time. This information can be printed using a specific layout on the medication sachet, either directly on a surface of the medication sachet or on a label that is attached on the medication sachet. The processing means may comprise a processor and a memory including computer program code, the memory and the computer program code being configured to, with the processor, determine patient and medication related information of the medication sachet.

According to an embodiment of the invention the arrangement comprises means for processing the at least one image captured with the second imaging device to detect a seam area between medication sachets. By a seam area is meant an area between two medication sachets along which the strip should be cut to separate the medication sachets from each other. The processing means may comprise a processor and a memory including computer program code, the memory and the computer program code being configured to, with the processor, detect a seam area between medication sachets.

According to an embodiment of the invention the means for conveying the medication sachets from the inlet to the outlet is arranged to simultaneously convey at least three medication sachets. The means for conveying the medication sachets from the inlet to the outlet can be arranged to simultaneously convey, for example, 3-5, 5-10, or 3-15 medication sachets.

According to an embodiment of the invention the means for conveying the medication sachets from the inlet to the outlet comprises a first endless belt, a plurality of first guide elements arranged to guide the first endless belt along a first path, at least one of the first guide elements being a first guide roller, a second endless belt, a plurality of second guide elements arranged to guide the second endless belt along a second path, at least one of the second guide elements being a second guide roller, a plurality of third guide rollers arranged to guide the first endless belt and the second endless belt along a third path, wherein the medication sachets are to be conveyed between the first endless belt and the second endless belt along the third path from the inlet to the outlet, and means for rotating at least one of the first, second or third guide rollers.

The first guide elements are in contact with the first endless belt and arranged to guide the first endless belt along the first path. The first guide elements can be, for example, guide rollers, slide bars, or slide surfaces. Preferably, all the first guide elements are first guide rollers. The second guide elements are in contact with the second endless belt and arranged to guide the second endless belt along the second path. The second guide elements can be, for example, guide rollers, slide bars, or slide surfaces. Preferably, all the second guide elements are second guide rollers. The third guide rollers are arranged to guide the first endless belt and the second endless belt together along the third path. The first endless belt and the second endless belt are driven by rotating at least one of the first guide rollers, or at least one of the second guide rollers, or at least one of the third guide rollers with the rotating means. The other guide rollers are rotated by the moving first and second endless belts. Preferably, the rotating means is arranged to rotate at least one of the third guide rollers. This has the advantage of the first endless belt and the second endless belt automatically moving at the same speed without a need to synchronise their movements.

The medication sachets to be conveyed are received one after another into the device through the inlet. From the inlet the medication sachets are conveyed between the first endless belt and the second endless along the third path to the outlet. The medication sachets travel between the first endless belt and the second endless belt around the third guide rollers. Since the medication sachets are encapsulated between the first endless belt and the second endless belt, their contact with the surrounding mechanics is prevented. The medication sachets are discharged out of the device through the outlet.

The number of the guide elements and rollers, and the paths of the endless belts can be chosen according to the space constraints and the desired length of the third path. The number of the first and second guide elements can be, for example, 4-15, and the number of the third guide rollers can be, for example, 2-8. The diameter of the first and second guide rollers can be, for example, 0.5-5 cm, and the diameter of the third guide rollers can be, for example, 2-20 cm. The guide elements and rollers can be made of, for example, aluminium, steel, or polymer. The first endless belt and the second endless belt can be made of, for example, silicone, polyurethane or other elastic material with favourable friction properties.

The conveying device may comprise a housing or a frame to which the guide elements and rollers, and the rotating means are supported. The housing or the frame can be made of, for example, aluminium, steel, or polymer.

According to an embodiment of the invention the means for rotating at least one of the first, second or third guide rollers comprises an electric motor coupled to said at least one guide roller. The electric motor may be directly coupled to an end of a shaft of one of the guide rollers. Alternatively, or in addition, the electric motor may be coupled to an end of a shaft of one or more guide rollers with gear wheels, or with cogged belt pulleys and cogged belts. Preferably, the electric motor is coupled to at least one of the third guide rollers.

According to an embodiment of the invention the conveying device comprises an inlet roller arranged at the inlet in connection with one of the plurality of first guide elements. Preferably, said first guide element is a first guide roller. The medication sachets are received into the conveying device between the inlet roller and said first guide element. The inlet roller is preferably free-rotating. The inlet roller can be suspended to the conveying device with one or more springs or rubber bands, which pull the inlet roller toward the first guide element. The spring or the rubber band can be attached between a shaft of the inlet roller and a housing or a frame of the conveying device. The diameter of the inlet roller can be, for example, 2-10 cm. An advantage of the inlet roller is that together with the first guide element it facilitates the receiving of the medication sachets into the conveying device.

According to an embodiment of the invention the inlet roller is arranged to be moveable with respect to the first guide element in connection with the inlet roller so that the distance between the inlet roller and said first guide element can vary. This enables medication sachets having different thicknesses to easily travel between the inlet roller and the first guide element.

According to an embodiment of the invention the conveying device comprises means for applying a force pulling the inlet roller toward the first guide element in connection with the inlet roller. This enables keeping the medication sachets in tight and continuous contact when they travel between the inlet roller and the first guide element, irrespective of their thicknesses. The means for applying a force may comprise one or more springs or rubber bands attached to a shaft of the inlet roller.

According to an embodiment of the invention the conveying device comprises an outlet roller arranged at the outlet in connection with one of the plurality of first guide elements. Preferably, said first guide element is a first guide roller. The medication sachets are discharged out of the conveying device between the outlet roller and said first guide element. The outlet roller is preferably free-rotating. The outlet roller can be suspended to the conveying device with one or more springs or rubber bands, which pull the outlet roller toward the first guide element. The spring or the rubber band can be attached between a shaft of the outlet roller and a housing or a frame of the conveying device. The diameter of the outlet roller can be, for example, 2-10 cm. An advantage of the outlet roller is that together with the first guide element it facilitates the discharging of the medication sachets out of the conveying device.

According to an embodiment of the invention the outlet roller is arranged to be moveable with respect to the first guide element in connection with the outlet roller so that the distance between the outlet roller and said first guide element can vary. This enables medication sachets having different thicknesses to easily travel between the outlet roller and the first guide element.

According to an embodiment of the invention the conveying device comprises means for applying a force pulling the outlet roller toward the first guide element in connection with the outlet roller. This enables keeping the medication sachets in tight and continuous contact when they travel between the outlet roller and the first guide element, irrespective of their thicknesses. The means for applying a force may comprise one or more springs or rubber bands attached to a shaft of the outlet roller.

According to an embodiment of the invention the inlet roller and/or the outlet roller are foam rollers. The elasticity of the foam roller provides a good grip and enables medication sachets having different thicknesses to easily travel between the foam roller and the first guide element. When foam rollers are used, the inlet and outlet rollers do not have to be moveable with respect to the first guide element. The foam rollers can be made of, for example, rubber, polyurethane foam, EPDM, or EVA.

According to an embodiment of the invention the length of the third path is 10-100 cm. Preferably, the length of the third path is 15-70 cm. Considering the medication sachets, this means that the conveying device can simultaneously convey 2-10 medication sachets between the first endless belt and the second endless belt.

According to an embodiment of the invention the number of the third guide rollers is 2-8. Preferably, the number of the third guide rollers is 2-5.

According to an embodiment of the invention the diameter of the third guide rollers is 2-20 cm. Preferably, the diameter of the third guide rollers is 5-15 cm.

According to an embodiment of the invention the third guide rollers have a larger diameter at the centre than at the ends. An advantage of this is that it reduces the stress on the strip of medication sachets because the shape enables the edges of the strip to take a slight shortcut around the third guide rollers. Without this shape there is a risk that the edges of the strip could be damaged. The diameter of the third guide roller can be, for example, 2-15 mm larger at the centre than at the ends. Preferably, the third guide rollers are barrel-shaped.

According to an embodiment of the invention the width of the first endless belt and the second endless belt is 4-15 cm. Preferably, the width of the first endless belt and the second endless belt is 6-10 cm. The width of the first endless belt and the second endless belt is preferably chosen in such a manner that it is at least 2 mm larger than the width of a medication sachet.

According to an embodiment of the invention the first endless belt and the second endless belt are made of an elastic material. The elastic material enables medication sachets having different thicknesses to be easily conveyed between the first endless belt and the second endless belt without requiring the use of soft guide rollers or moving shafts. The elastic material can be, for example, silicone, polyurethane or other elastic material with favourable friction properties.

According to an embodiment of the invention the conveying device comprises a chamber for temporarily storing a plurality of medication sachets. The medication sachets are stored in the chamber in a FIFO principle. The chamber may have a capacity of temporarily storing, for example, 3-20 medication sachets.

According to an embodiment of the invention the arrangement comprises a pair of rollers arranged in connection with the first imaging device and configured to convey the medication sachets to the first imaging device.

According to an embodiment of the invention the first and/or the second imaging device comprises a first polarizer and a second polarizer between which a strip of medication sachets can be arranged, a camera for imaging a first side of the strip of medication sachets through the second polarizer, and means for changing the polarization direction of the first polarizer or the second polarizer.

The first polarizer and the second polarizer of the imaging device are linear polarizers. A linear polarizer is an optical filter that allows light waves of a specific polarization to pass through while blocking light waves of other polarizations. In other words, the linear polarizer works by transmitting light that oscillates in a particular direction (the polarization direction) and absorbing or reflecting light that oscillates in other directions. The purpose of the first polarizer and the second polarizer is to enhance the visibility of a seam area (heat seal). The polarization of the light on the seam area differs from that on the other areas. The first polarizer and the second polarizer are arranged in parallel and at a distance from each other. The distance between the first polarizer and the second polarizer can be, for example, 10-150 mm. A strip of medication sachets to be imaged is meant to be conveyed between the first polarizer and the second polarizer so that a first side of the strip faces a first side of the second polarizer, and a second side of the strip faces a first side of the first polarizer.

The polarization direction of the first polarizer or the second polarizer can be changed with the means for changing the polarization direction. The polarization direction of the polarizer can be electrically or mechanically controllable. The means for changing the polarization direction of the first polarizer or the second polarizer may comprise an actuator for mechanically controlling the polarization direction by rotating the polarizer, or a control unit that is configured to electrically control the polarization direction by changing the electrical voltage or current that is supplied to the polarizer.

The camera is arranged to image the first side of the strip of medication sachets through the second polarizer. The camera is configured to capture images of the strip using different polarization directions of the first or the second polarizer. The camera is preferably a digital camera. One or more lenses and/or mirrors can be arranged in an optical path between the camera and the second polarizer.

According to an embodiment of the invention the means for changing the polarization direction of the first polarizer or the second polarizer is configured to change the polarization direction between a first polarization direction, where the polarization directions of the first polarizer and the second polarizer are perpendicular, and a second polarization direction, where the polarization directions of the first polarizer and the second polarizer are parallel.

When the polarization directions of the first polarizer and the second polarizer are perpendicular, the polarizers can block the passage of light from a second side of the first polarizer to the second side of the second polarizer, unless the polarization state of the light has changed due to interaction with the strip of medication sachets. The areas where the polarization has changed appear lighter in the image. When the polarization directions of the first polarizer and the second polarizer are parallel, the polarizers allow light that oscillates in the second polarization direction to pass through the polarizers from the second side of the first polarizer to the second side of the second polarizer. If the polarization state of the light has changed due to interaction with the strip of medication sachets, these areas appear darker in the image.

The camera is preferably configured to capture images of the strip of medication sachets both in the case where the polarization directions of the first polarizer and the second polarizer are perpendicular and where the polarization directions of the first polarizer and the second polarizer are parallel. An advantage of this is that it improves the detection of a seam area between medication sachets.

According to an embodiment of the invention the first and/or the second imaging device comprises a first light source for illuminating a second side of the strip of medication sachets through the first polarizer. The first light source is arranged at the second side of the first polarizer. The amount and polarization state of the light received into the camera from the first light source depends on the polarization directions of the first polarizer and the second polarizer as well as the properties of the strip of medication sachets through which the light passes.

The first light source is preferably arranged to provide an essentially even light distribution on the second side of the strip. The first light source can be a light panel that is arranged in contact with or at a distance from the second side of the first polarizer. The first light source may comprise one or more LEDs. The LEDs may be arranged together in an LED array or matrix. The imaging device may comprise a control unit that is configured to switch the first light source on and off.

According to an embodiment of the invention the first and/or the second imaging device comprises a second light source for illuminating the first side of the strip of medication sachets. The second light source is preferably arranged in a space between the first polarizer and the second polarizer. Part of the light emitted by the second light source is reflected from the first side of the strip of medication sachets and passed through the second polarizer into the camera. The amount and polarization state of the light received into the camera from the second light source depends on the polarization direction of the second polarizer as well as the properties of the strip of medication sachets.

The second light source is preferably arranged to provide an essentially even light distribution on the first side of the strip. The second light source may comprise one or more LEDs. The LEDs may be arranged together in an LED array or matrix. The imaging device may comprise a control unit that is configured to switch the second light source on and off.

According to an embodiment of the invention the first and/or the second imaging device comprises a reflector for reflecting light from the second light source towards the first side of the strip of medication sachets, the reflector having an aperture through which the first side of the strip of medication sachets can be imaged. The reflector is preferably arranged in a space between the first polarizer and the second polarizer. The reflector is arranged in such a manner that its opening faces the first side of the first polarizer. The reflector is preferably shaped so that together with the second light source they provide an essentially even light distribution on the first side of the strip of medication sachets. The aperture through which the strip of medication sachets is imaged faces the first side of the second polarizer. The aperture can be, for example, circular or rectangular in shape. The diameter of a circular aperture can be, for example, 5-30 mm, and the length of each side of a rectangular aperture can be, for example, 5-30 mm.

According to an embodiment of the invention the reflector is a tunnel-shaped reflector. The strip of medication sachets can be conveyed inside the tunnel-shaped reflector.

According to an embodiment of the invention the second light source is attached to an opening of the reflector. The second light source is preferably attached to the opening of the reflector in such a manner that a main part of the light emitted by the second light source is directed towards a reflecting surface of the reflector from which reflecting surface the light is reflected towards the first side of the strip of medication sachets.

According to an embodiment of the invention the first and/or the second imaging device comprises a transparent panel for supporting the strip of medication sachets. The transparent panel is arranged in a space between the first polarizer and the second polarizer to support the second side of the strip of medication sachets. The transparent panel can be made of glass or acrylic.

The present invention also relates to a medication dispenser for dispensing medications to a patient. The medication dispenser comprises an arrangement according to the invention for handling a strip of medication sachets.

The medication dispenser may comprise a chamber into which a strip of medication sachets can be inserted. The strip of medication sachets is conveyed from the chamber through the first imaging device, the conveying device and the second imaging device to an outlet of the medication dispenser. The medication dispenser separates the medication sachets from the strip and dispenses them to the patient one medication sachet at a time. For this purpose, the medication dispenser may comprise a cutting device that is configured to separate the medication sachets from the strip of medication sachets by cutting the strip in a transverse direction along seam areas between the medication sachets. The separated medication sachets can be conveyed to the outlet from which the patient can take the medication sachets.

According to an embodiment of the invention the medication dispenser comprises a cutting device arranged in connection with the second imaging device. The cutting device can be arranged between the second imaging device and the outlet of the conveying device. The cutting device is used for separating the medication sachets from the strip of medication sachets by cutting the strip in a transverse direction along the detected seam areas.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

### BRIEF DESCRIPTION OF THE DRAWINGS

- Fig. 1: illustrates an arrangement according to an embodiment of the invention,
- fig. 2: illustrates a medication dispenser according to an embodiment of the invention,
- fig. 3: illustrates an example of an imaging device,
- fig. 4: illustrates another example of an imaging device, and
- fig. 5: illustrates an example of a conveying device.

### DETAILED DESCRIPTION OF THE DRAWINGS

The same reference signs are used of the same or like components in different embodiments.

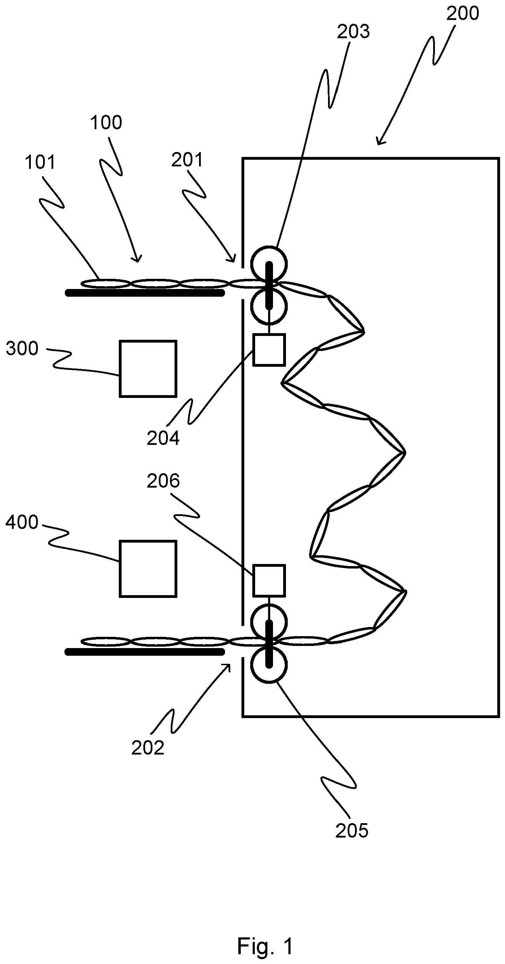

Fig. 1 illustrates an arrangement according to an embodiment of the invention. The arrangement is used for handling a strip 100 of medication sachets 101. Each medication sachet 101 contains the medications that a patient should take at a prescribed date and time.

The arrangement comprises a conveying device 200 and two imaging devices 300 and 400. The imaging device 300 is arranged in connection with an inlet 201 of the conveying device 200 and configured to capture at least one image of the medication sachet 101 before it is conveyed into the conveying device 200 through the inlet 201. The imaging device 300 comprises a processing unit (not shown in fig. 1) that is configured to process the captured image(s) to determine patient and medication related information of the medication sachet 101. The patient and medication related information may contain a patient identifier (e.g. name and/or identification number), and dispense date and time. This information enables detecting situations where medication sachets 101 are running out, or a wrong strip 100 of medication sachets 101 has been inserted into a medication dispenser. The processing unit may also be configured to process the captured image(s) to detect an end of the strip 100. The imaging device 400 is arranged in connection with an outlet 202 of the conveying device 200 and configured to capture at least one image of the medication sachet 101 after it has been conveyed out of the conveying device 200 through the outlet 202. The imaging device 400 comprises a processing unit (not shown in fig. 1) that is configured to process the captured image(s) to detect a seam area between two medication sachets 101.

The conveying device 200 comprises a pair of rollers 203 at the inlet 201 for conveying the medication sachets 101 into the conveying device 200. The rollers 203 are driven by an electric motor 204. The conveying device 200 comprises another pair of rollers 205 at the outlet 202 for conveying the medication sachets 101 out of the conveying device 200. The rollers 205 are driven by an electric motor 206. The amount of medication sachets 101 inside the conveying device 200 can be changed by controlling the operation of the electric motors 204 and 206.

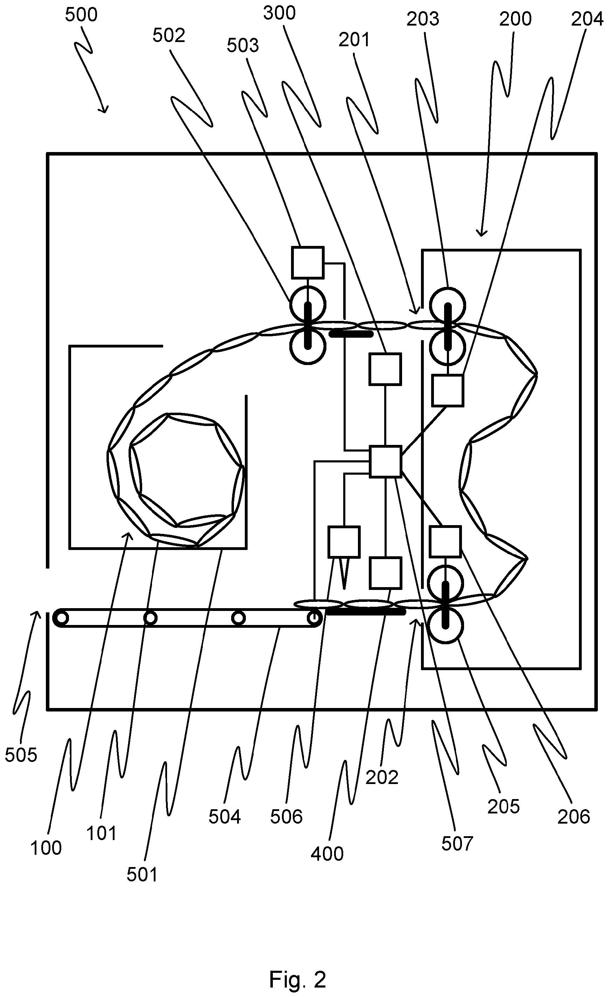

Fig. 2 illustrates a medication dispenser according to an embodiment of the invention for dispensing medications to a patient. The medication dispenser 500 comprises a chamber 501 into which a strip 100 of medication sachets 101 has been inserted. The medication dispenser 500 comprises a pair of rollers 502 driven by an electric motor 503 for conveying the medication sachets 101 from the chamber 501 to an inlet 201 of a conveying device 200. The conveying device 200 is similar to the one shown in fig. 1. The medication sachets 101 are conveyed into the conveying device 200 through the inlet 201 by using a pair of rollers 203. The rollers 203 are driven by an electric motor 204. The medication sachets 101 are conveyed out of the conveying device 200 through an outlet 202 by using a pair of rollers 205. The rollers 205 are driven by an electric motor 206. The medication dispenser 500 comprises a belt conveyor 504 for conveying the medication sachets 101 from the outlet 202 to an outlet 505 of the medication dispenser 500.

The medication dispenser 500 comprises an imaging device 300 that is arranged in connection with the inlet 201. The imaging device 300 is configured to capture at least one image of the medication sachet 101 before it is conveyed to the inlet 201 and to process the captured image(s) to determine patient and medication related information of the medication sachet 101. The medication dispenser 500 comprises another imaging device 400 that is arranged in connection with the outlet 202. The imaging device 400 is configured to capture at least one image of the medication sachet 101 after it has been conveyed out of the outlet 202 and to process the captured image(s) to detect a seam area between two medication sachets 101.

The medication dispenser 500 comprises a cutting device 506 that is arranged between the imaging device 400 and the belt conveyor 504. The cutting device 506 is configured to cut the strip 100 in a transverse direction at the seam areas that have been detected with the imaging device 400. The separated medication sachets 101 are conveyed with the belt conveyor 504 to the outlet 505 from which the patient can take the medication sachets 101.

The medication dispenser 500 comprises a control unit 507 that is connected to and configured to control the operation of the electric motors 204, 206 and 503, the imaging devices 300 and 400, the belt conveyor 504, and the cutting device 506.

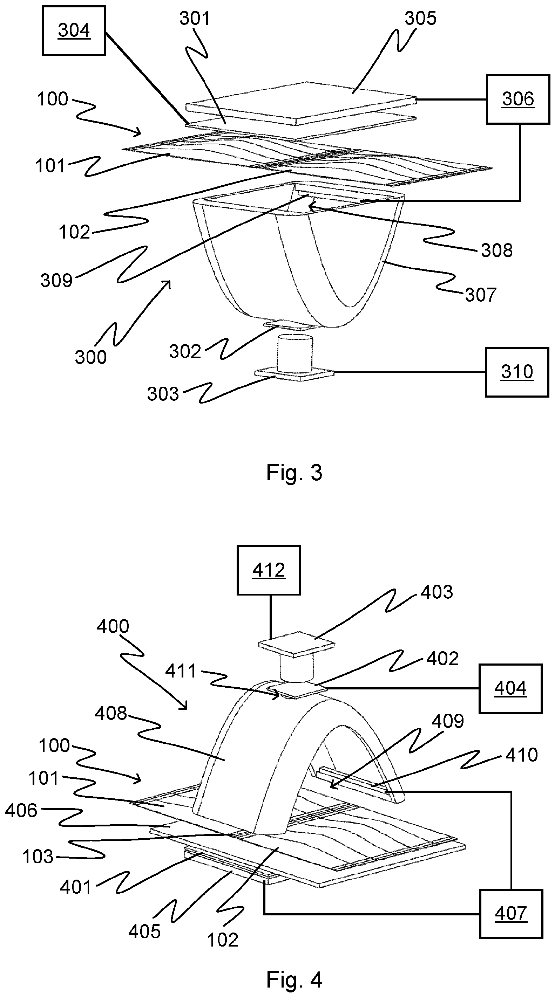

Fig. 3 illustrates an example of an imaging device that can be arranged in connection with an inlet of a conveying device. The imaging device 300 comprises two polarizers 301 and 302 between which a strip 100 that consists of two medication sachets 101 and 102 is arranged, and a camera 303 for imaging a lower side of the strip 100 through the polarizer 302. The imaging device 300 comprises a control unit 304 that is connected to the polarizer 301 and configured to change the polarization direction of the polarizer 301 between a first polarization direction, where the polarization directions of the polarizers 301 and 302 are perpendicular, and a second polarization direction, where the polarization directions of the polarizers 301 and 302 are parallel.

The imaging device 300 comprises a light source 305 that is arranged at a distance from an upper side of the polarizer 301. The light source 305 is used for illuminating an upper side of the strip 100 through the polarizer 301. The amount and polarization state of the light received into the camera 303 from the light source 305 depends on the polarization directions of the polarizers 301 and 302 as well as the properties of the strip 100 through which the light passes. The imaging device 300 comprises a control unit 306 that is connected to the light source 305 and configured to switch the light source 305 on and off.

The imaging device 300 comprises a reflector 307 that is arranged in a space between the polarizers 301 and 302 in such a manner that its opening 308 faces the lower side of the strip 100, and a light source 309 that is attached to the opening 308 of the reflector 307. The reflector 307 is used for reflecting light from the light source 309 towards the lower side of the strip 100. Part of the light is reflected from the lower side of the strip 100 and passed through the polarizer 302 into the camera 303. The reflector 307 comprises an aperture (not shown in fig. 3) through which the lower side of the strip 100 is imaged. The amount and polarization state of the light received into the camera 303 from the light source 309 depends on the polarization direction of the polarizer 302 as well as the properties of the strip 100. The control unit 306 is connected to the light source 309 and configured to switch the light source 309 on and off.

The camera 303 is configured to capture images of the strip 100 using different polarization directions of the polarizer 301 and different settings of the light sources 305 and 309. The imaging device 300 comprises a processing unit 310 that is connected to the camera 303 and configured to process the captured images to determine patient and medication related information of the medication sachets 101 and 102.

Fig. 4 illustrates another example of an imaging device that can be arranged in connection with an outlet of a conveying device. The imaging device 400 comprises two polarizers 401 and 402 between which a strip 100 that consists of two medication sachets 101 and 102 is arranged, and a camera 403 for imaging an upper side of the strip 100 through the polarizer 402. The imaging device 400 comprises a control unit 404 that is connected to the polarizer 402 and configured to change the polarization direction of the polarizer 402 between a first polarization direction, where the polarization directions of the polarizers 401 and 402 are perpendicular, and a second polarization direction, where the polarization directions of the polarizers 401 and 402 are parallel.

The imaging device 400 comprises a light source 405 that is arranged in contact with a lower side of the polarizer 401. The light source 405 is used for illuminating a lower side of the strip 100 through the polarizer 401. The amount and polarization state of the light received into the camera 403 from the light source 405 depends on the polarization directions of the polarizers 401 and 402 as well as the properties of the strip 100 and a transparent panel 406 through which the light passes. The transparent panel 406 is used for supporting the strip 100. The imaging device 400 comprises a control unit 407 that is connected to the light source 405 and configured to switch the light source 405 on and off.

The imaging device 400 comprises a reflector 408 that is arranged in a space between the polarizers 401 and 402 in such a manner that its opening 409 faces the upper side of the strip 100, and a light source 410 that is attached to the opening 409 of the reflector 408. The reflector 408 is used for reflecting light from the light source 410 towards the upper side of the strip 100. Part of the light is reflected from the upper side of the strip 100 and passed through the polarizer 402 into the camera 403. The reflector 408 comprises an aperture 411 through which the upper side of the strip 100 is imaged. The amount and polarization state of the light received into the camera 403 from the light source 410 depends on the polarization direction of the polarizer 402 as well as the properties of the strip 100. The control unit 407 is connected to the light source 410 and configured to switch the light source 410 on and off.

The camera 403 is configured to capture images of the strip 100 using different polarization directions of the polarizer 402 and different settings of the light sources 405 and 410. The imaging device 400 comprises a processing unit 412 that is connected to the camera 403 and configured to process the captured images to detect the location of a seam area 103 between the medication sachets 101 and 102.

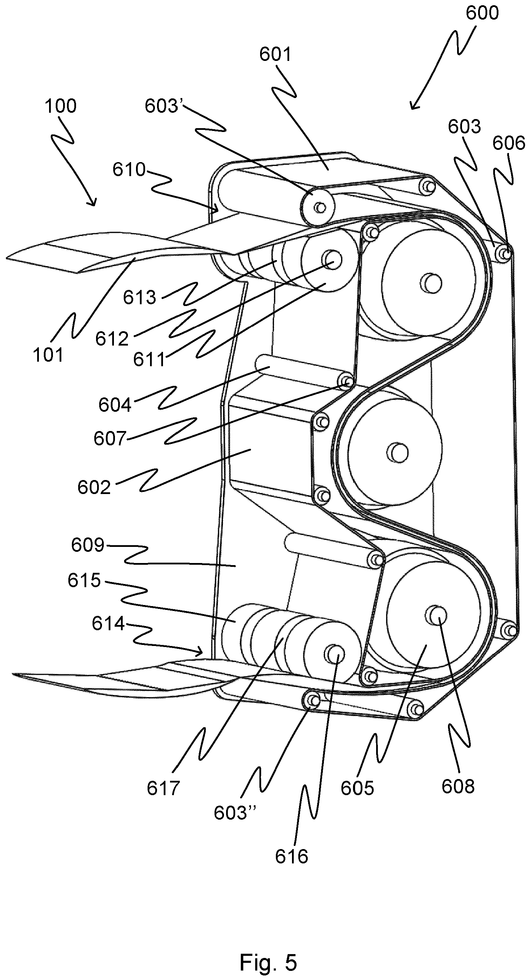

Fig. 5 illustrates an example of a conveying device for conveying a strip of medication sachets. The conveying device 600 comprises two endless belts 601 and 602, and three sets of guide rollers 603, 604 and 605. The guide rollers 603 are arranged to guide the endless belt 601 along a first path, and the guide rollers 604 are arranged to guide the endless belt 602 along a second path. The guide rollers 605 are arranged to guide the endless belts 601 and 602 together along a third path. The endless belts 601 and 602 are driven by rotating the top and bottom guide rollers 605 with a drive system (not shown in fig. 5). The middle guide roller 605 and the other guide rollers 603 and 604 are rotated by the moving endless belts 601 and 602. The guide rollers 603, 604 and 605 are supported at their shafts 606, 607 and 608 between two side plates 609 of which only one can be seen in fig. 5.

The medication sachets 101 to be conveyed are received one after another into the conveying device 600 through an inlet 610. At the inlet 610, there is an inlet roller 611 that is arranged in connection with the guide roller 603'. The medication sachets 101 are received into the conveying device 600 between the inlet roller 611 and the guide roller 603'. The inlet roller 611 is supported at its shaft 612 between the side plates 609. The inlet roller 611 has grooves 613. The inlet roller 611 is made of a foam material, which provides a good grip and enables medication sachets 101 having different thicknesses to easily travel between the inlet roller 611 and the guide roller 603'.

From the inlet 610 the medication sachets 101 are conveyed between the endless belts 601 and 602 around the guide rollers 605 to an outlet 614. Since the medication sachets 101 are encapsulated between the endless belts 601 and 602, their contact with the surrounding mechanics is prevented. The guide rollers 605 have a larger diameter at the centre than at the ends. This shape reduces the stress on the strip 100 because the edges of the strip 100 can take a slight shortcut around the guide rollers 605. The endless belts 601 and 602 are made of an elastic material, which enables medication sachets 101 having different thicknesses to be easily conveyed between the endless belts 601 and 602.

The medication sachets 101 are delivered out of the conveying device 600 through the outlet 614. At the outlet 614, there is an outlet roller 615 that is arranged in connection with the guide roller 603". The medication sachets 101 are delivered out of the conveying device 600 between the outlet roller 615 and the guide roller 603". The outlet roller 615 is supported at its shaft 616 between the side plates 609. The outlet roller 615 has grooves 617. The outlet roller 615 is made of a foam material, which provides a good grip and enables medication sachets 101 having different thicknesses to easily travel between the outlet roller 615 and the guide roller 603".

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. An arrangement for handling a strip of medication sachets, comprising:
- a conveying device for conveying the strip of medication sachets, the conveying device comprising an inlet for receiving the medication sachets, an outlet for discharging the medication sachets and means for conveying the medication sachets from the inlet to the outlet,
**characterised in that** the arrangement comprises:
- a first imaging device arranged in connection with the inlet and configured to capture at least one image of the medication sachet before it is conveyed to the inlet, and
- a second imaging device arranged in connection with the outlet and configured to capture at least one image of the medication sachet after it has been conveyed out of the outlet.

2. The arrangement according to claim 1, **characterised in that** the arrangement comprises means for processing the at least one image captured with the first imaging device to determine patient and medication related information of the medication sachet.

3. The arrangement according to claim 1 or 2, **characterised in that** the arrangement comprises means for processing the at least one image captured with the second imaging device to detect a seam area between medication sachets.

4. The arrangement according to any of the preceding claims, **characterised in that** the means for conveying the medication sachets from the inlet to the outlet is arranged to simultaneously convey at least three medication sachets.

5. The arrangement according to any of the preceding claims, **characterised in that** the means for conveying the medication sachets from the inlet to the outlet comprises:
- a first endless belt,
- a plurality of first guide elements arranged to guide the first endless belt along a first path, at least one of the first guide elements being a first guide roller,
- a second endless belt,
- a plurality of second guide elements arranged to guide the second endless belt along a second path, at least one of the second guide elements being a second guide roller,
- a plurality of third guide rollers arranged to guide the first endless belt and the second endless belt along a third path, wherein the medication sachets are to be conveyed between the first endless belt and the second endless belt along the third path from the inlet to the outlet, and
- means for rotating at least one of the first, second or third guide rollers.

6. The arrangement according to claim 5, **characterised in that** the means for rotating at least one of the first, second or third guide rollers comprises an electric motor coupled to said at least one guide roller.

7. The arrangement according to claim 5 or 6, **characterised in that** the conveying device comprises an inlet roller arranged at the inlet in connection with one of the plurality of first guide elements.

8. The arrangement according to any of claims 5 to 7, **characterised in that** the conveying device comprises an outlet roller arranged at the outlet in connection with one of the plurality of first guide elements.

9. The arrangement according to any of the preceding claims, **characterised in that** the conveying device comprises a chamber for temporarily storing a plurality of medication sachets.

10. The arrangement according to any of the preceding claims, **characterised in that** the first and/or the second imaging device comprises:
- a first polarizer and a second polarizer between which a strip of medication sachets can be arranged,
- a camera for imaging a first side of the strip of medication sachets through the second polarizer, and
- means for changing the polarization direction of the first polarizer or the second polarizer.

11. The arrangement according to claim 10, **characterised in that** the means for changing the polarization direction of the first polarizer or the second polarizer is configured to change the polarization direction between a first polarization direction, where the polarization directions of the first polarizer and the second polarizer are perpendicular, and a second polarization direction, where the polarization directions of the first polarizer and the second polarizer are parallel.

12. The arrangement according to claim 10 or 11, **characterised in that** the first and/or the second imaging device comprises a first light source for illuminating a second side of the strip of medication sachets through the first polarizer.

13. The arrangement according to any of claims 10 to 12, **characterised in that** the first and/or the second imaging device comprises a second light source for illuminating the first side of the strip of medication sachets.

14. The arrangement according to claim 13, **characterised in that** the first and/or the second imaging device comprises a reflector for reflecting light from the second light source towards the first side of the strip of medication sachets, the reflector having an aperture through which the first side of the strip of medication sachets can be imaged.

15. A medication dispenser, **characterised in that** the medication dispenser comprises an arrangement according to any of the preceding claims for handling a strip of medication sachets.

## Amended claims

### Amended claims in accordance with Rule 137(2) EPC.

1. An arrangement for handling a strip (100) of medication sachets (101), comprising:
- a conveying device (200, 600) for conveying the strip (100) of medication sachets (101), the conveying device (200, 600) comprising an inlet (201, 610) for receiving the medication sachets (101), an outlet (202, 614) for discharging the medication sachets (101) and means (203, 204, 205, 206, 601, 602, 603, 604, 605) for conveying the medication sachets (101) from the inlet (201, 610) to the outlet (202, 614),
- a first imaging device (300) arranged in connection with the inlet (201, 610) and configured to capture at least one image of the medication sachet (101) before it is conveyed to the inlet (201, 610), and
- a second imaging device (400) arranged in connection with the outlet (202, 614) and configured to capture at least one image of the medication sachet (101) after it has been conveyed out of the outlet (202, 614),
**characterised in that** the means (203, 204, 205, 206, 601, 602, 603, 604, 605) for conveying the medication sachets (101) from the inlet (201, 610) to the outlet (202, 614) comprises:
- a first endless belt (601),
- a plurality of first guide elements (603) arranged to guide the first endless belt (601) along a first path, at least one of the first guide elements (603) being a first guide roller (603),
- a second endless belt (602),
- a plurality of second guide elements (604) arranged to guide the second endless belt (602) along a second path, at least one of the second guide elements (604) being a second guide roller (604),
- a plurality of third guide rollers (605) arranged to guide the first endless belt (601) and the second endless belt (602) along a third path, wherein the medication sachets (101) are to be conveyed between the first endless belt (601) and the second endless belt (602) along the third path from the inlet (610) to the outlet (614), and
- means for rotating at least one of the first, second or third guide rollers (603, 604, 605).

2. The arrangement according to claim 1, **characterised in that** the arrangement comprises means (310) for processing the at least one image captured with the first imaging device (300) to determine patient and medication related information of the medication sachet (101).

3. The arrangement according to claim 1 or 2, **characterised in that** the arrangement comprises means (412) for processing the at least one image captured with the second imaging device (400) to detect a seam area (103) between medication sachets (101, 102).

4. The arrangement according to any of the preceding claims, **characterised in that** the means (203, 204, 205, 206, 601, 602, 603, 604, 605) for conveying the medication sachets (101) from the inlet (201, 610) to the outlet (202, 614) is arranged to simultaneously convey at least three medication sachets (101).

5. The arrangement according to any of the preceding claims, **characterised in that** the means for rotating at least one of the first, second or third guide rollers (603, 604, 605) comprises an electric motor coupled to said at least one guide roller (603, 604, 605).

6. The arrangement according to any of the preceding claims, **characterised in that** the conveying device (200, 600) comprises an inlet roller (611) arranged at the inlet (610) in connection with one of the plurality of first guide elements (603').

7. The arrangement according to any of the preceding claims, **characterised in that** the conveying device (200, 600) comprises an outlet roller (615) arranged at the outlet (614) in connection with one of the plurality of first guide elements (603").

8. The arrangement according to any of the preceding claims, **characterised in that** the conveying device (200, 600) comprises a chamber for temporarily storing a plurality of medication sachets (101).

9. The arrangement according to any of the preceding claims, **characterised in that** the first and/or the second imaging device (300, 400) comprises:
- a first polarizer (301, 401) and a second polarizer (302, 402) between which a strip (100) of medication sachets (101) can be arranged,
- a camera (303, 403) for imaging a first side of the strip (100) of medication sachets (101) through the second polarizer (302, 402), and
- means (304, 404) for changing the polarization direction of the first polarizer (301, 401) or the second polarizer (302, 402).

10. The arrangement according to claim 9, **characterised in that** the means (304, 404) for changing the polarization direction of the first polarizer (301, 401) or the second polarizer (302, 402) is configured to change the polarization direction between a first polarization direction, where the polarization directions of the first polarizer (301, 401) and the second polarizer (302, 402) are perpendicular, and a second polarization direction, where the polarization directions of the first polarizer (301, 401) and the second polarizer (302, 402) are parallel.

11. The arrangement according to claim 9 or 10, **characterised in that** the first and/or the second imaging device (300, 400) comprises a first light source (305, 405) for illuminating a second side of the strip (100) of medication sachets (101) through the first polarizer (301, 401).

12. The arrangement according to any of claims 9 to 11, **characterised in that** the first and/or the second imaging device (300, 400) comprises a second light source (309, 410) for illuminating the first side of the strip (100) of medication sachets (101).

13. The arrangement according to claim 12, **characterised in that** the first and/or the second imaging device (300, 400) comprises a reflector (307, 408) for reflecting light from the second light source (309, 410) towards the first side of the strip (100) of medication sachets (101), the reflector (307, 408) having an aperture (411) through which the first side of the strip (100) of medication sachets (101) can be imaged.

14. A medication dispenser (500), **characterised in that** the medication dispenser (500) comprises an arrangement according to any of the preceding claims for handling a strip (100) of medication sachets (101).
